(19) Europäisches Patentamt
European Patent Office
Office européen des brevets

(11) Veröffentlichungsnummer: **0 095 557**
**B1**

# (12) EUROPÄISCHE PATENTSCHRIFT

(45) Veröffentlichungstag der Patentschrift: **27.12.85**

(51) Int. Cl.[4]: **C 07 K 7/06**, C 07 C 102/00, A 61 K 37/02

(21) Anmeldenummer: **83101523.5**

(22) Anmeldetag: **18.02.83**

(54) **Polypeptide mit antagonistischen Eigenschaften gegenüber der Substanz P, Verfahren zu ihrer Herstellung, deren Verwendung und Verfahren zum Reinigen von Polypeptiden.**

(30) Priorität: **19.02.82 DE 3205991**

(43) Veröffentlichungstag der Anmeldung: **07.12.83 Patentblatt 83/49**

(45) Bekanntmachung des Hinweises auf die Patenterteilung: **27.12.85 Patentblatt 85/52**

(84) Benannte Vertragsstaaten: **AT CH DE FR GB IT LI NL SE**

(56) Entgegenhaltungen: **EUROPEAN JOURNAL OF PHARMACOLOGY, Band 77, Nr. 2-3, 1982, CARANIKAS et al. "Antagonists of substance P", Seiten 205-206**

(73) Patentinhaber: **Ferring Arzneimittel GmbH, Wittland 11, D-2300 Kiel 1 (DE)**

(72) Erfinder: **Hörig, Joachim, Dr. Dipl.-Ern., Vollbehrstrasse 16a, D-2300 Kronshagen (DE)**
Erfinder: **Schultheiss, Hartmut, Dr. Dipl.-biol., Ulmenstrasse 1, D-2301 Raisdorf (DE)**

(74) Vertreter: **Türk, Dietmar, Dr. rer. nat. et al, Patentanwälte Türk & Gille Brucknerstrasse 20, D-4000 Düsseldorf 13 (DE)**

**EP 0 095 557 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Polypeptide, die ausgeprägte antagonistische Eigenschaften gegenüber der Substanz P besitzen, ein Verfahren zu ihrer Herstellung, deren Verwendung bei der Bekämpfung von Krankheiten und sie enthaltende pharmazeutische Präparate. Die Erfindung betrifft weiterhin ein vorteilhaftes Verfahren zur Reinigung von Polypeptiden.

Euler und Gaddum ((1931, J. Physiol. (London) 72, 74—87) beschrieben als erste eine biologische Aktivität aus Gewebsextrakten, die eine kontraktionsstimulierende Wirkung am Jejunum des Meerschweinchens und eine blutgefäßerweiternde Wirkung beim Kaninchen hatte. Sie nannten die Aktivität »Substanz P«, waren allerdings nicht in der Lage, die verantwortliche Verbindung in reiner Form zu isolieren. Dies gelang erst später, als Leeman und Hammerschlag 1967 (Endocrinology 81, 803—810) die speichelfördernde Wirkung einer Hypothalamusfraktion fanden. Infolge dieses einfachen Nachweissystems war die Arbeitsgruppe in der Lage, das wirksame Prinzip in homogener Form zu erhalten (Chang & Leeman 1979, J. Biol. Chem. 245, 4784—4790). Es handelt sich um ein Peptid der folgenden Struktur:

Arg-Pro-Lys-Pro-Gln-Gln-Phe-Phe-Gly-Leu-Met-$NH_2$

Dieses Peptid wurde bald als Substanz P identifiziert, da es dieselben Eigenschaften hatte wie die von Euler und Gaddum teilweise gereinigten Fraktionen. Da das Peptid jetzt synthetisch zugänglich war (die erste Synthese wurde von Tregear et al. (1974, Nature (London) 232, 87—89 beschrieben), konnte auch ein spezifischer Radioimmunoassay für das Peptid ausgearbeitet werden. Dieser erlaubte die Bestimmung der Verteilung der Substanz in verschiedenen Geweben und stimulierte damit die Substanz P-Forschung beachtlich.

Substanz P wurde in einer Vielzahl von verschiedenen Geweben und zellulären Strukturen nachgewiesen. Da dies in den meisten Fällen durch radioimmunologische Methoden geschah, wird in der Literatur von ISP (immunoreactive Substance P) gesprochen. Organe, in denen ISP vorkommt, sind: Säugetierdarm, Speicheldrüse, Trachea, Pankreas, Niere, Blase, Prostata, Substantia nigra, Hypothalamus, Zirbeldrüse, Dorsalhorn.

ISP ist offensichtlich in Nerven lokalisiert, die dem autonomen Nervensystem und primären sensorischen Nervenfasern (z. B. Fasern, die für die Schmerzweiterleitung verantwortlich sind) angehören. ISP wurde im Plexus myentericus (Auerbach'scher Plexus) und in verstreuten Nervenfasern zwischen den glatten Muskelzellen des Dünndarmes gefunden. Außerdem kommt ISP in endokrinen Zellen der Darmmucosa vor.

Ein großer Vorteil bei der Erforschung der biologischen Funktionen von Substanz P wäre ein Inhibitor, der spezifisch die Wirkung des Peptides blockiert.

Kürzlich gelang es, spezifische und kompetitive Inhibitoren zu synthetisieren. Es handelt sich hierbei um Substanzen, bei denen die Aminosäuren in den Positionen 2, 7 und 9 gegen andere Aminosäuren ausgetauscht sind. D-$Pro^2$, D-$Phe^7$, D-$Trp^9$-SP (Folkers et al. (1981) Acta Physiol. Scand. 111, 505—506; Rosell et al. (1981) Acta Physiol. Scand. 111, 381—382) inhibiert die Kontraktion des Meerschweinchenileums durch SP, die durch SP-induzierte Speichelsekretion und die durch antidrome Nervenreizung sowie durch SP-induzierte Vasodilatation.

Der vorliegenden Erfindung liegt die Aufgabenstellung zugrunde neue Polypeptide zu finden, die eine praktisch ausschließliche antagonistische Wirkung gegenüber Substanz P haben und als Arzneimittel für Menschen und Tiere zur Bekämpfung gewisser Krankheiten geeignet sind. Weiterhin liegt der Erfindung die Aufgabenstellung zugrunde, Polypeptide in einfacher und vorzüglicher Weise zu reinigen, so daß sie für den genannten Verwendungszweck einwandfrei eingesetzt werden können.

Gegenstand der vorliegenden Erfindung sind demgemäß Polypeptide der Formel

V-X-D-Trp-Y-D-Trp-Leu-Z-$NH_2$ (I)

wobei

V eine in der Natur vorkommende Aminosäure oder einen physiologisch unbedenklichen Säurerest,
X eine in der Natur vorkommenden Aminosäure oder eine einfache Bindung,
Y eine der Aminosäuren Phe, Ile, Val, Tyr und L-p-Cl-Phe, und
Z die Aminosäuren Met oder Nle

darstellen und deren Salze.

Das Verfahren zur Herstellung der genannten Polypeptide ist dadurch gekennzeichnet, daß man in an sich bekannter Weise nach der Festphasenmethode an ein Trägerharz zuerst die C-terminale Aminosäure und dann daran die anderen Aminosäuren bzw. die physiologisch unbedenkliche Säure, für die u. a. das Symbol V stehen kann, koppelt und das Trägerharz abspaltet. Die Festphasenmethode ist im

einzelnen beschrieben von Merrifield in (R. B. Merrifield (1963), J. Amer. Chem. Soc. 85, 2149–2154). Als Festphase wird zweckmäßig das dort beschriebene chlormethylierte Polystyrol (1 % quervernetzt) verwendet.

Bei der Festphasensynthese von Peptiden wird im allgemeinen zuerst die C-terminale Aminosäure kovalent an das Harz gebunden. Dies geschieht zweckmäßig durch Umsetzung der Cs-Salze der Boc-Aminosäuren mit den Chlormethylgruppen des Harzes. Es wird unter Austritt von CsCl eine Esterbindung zwischen Harz und Peptid geknüpft. Die Aminosäure darf außer der Carboxylgruppe keine weiteren, während der Kopplung reaktiven Gruppen tragen. Zur Blockierung der alpha-$NH_2$-Gruppe kann z. B. die t-Butyloxycarbonylgruppe (Boc) verwendet werden. An der an das Harz gekoppelten ersten C-terminalen Aminosäure wird dann das gesamte Peptid schrittweise aufgebaut.

Die t-Butyloxycarbonylgruppe (Boc) kann bei den Aminosäuren wie Pro, Gly, Phe, Ile, Leu, Nle, Met, D-Trp mit Hilfe von 2-(t-Butyloxycarbonyloxymino)-2-phenylacetonitril (Boc-ON) eingeführt werden (M. Itoh, D. Hagiwara, T. Kamiya (1975), Tetrahedron Letters 4 393).

D-Trp (For) kann z. B. nach M. Ohno, S. Tsukamoto, S. Makisuma, N. Izumiyu (1972) Bull. Chem. Soc. Jap. 45, 2852 hergestellt werden. Die Darstellung von Boc-Arg (HCl) · $H_2O$ kann z. B. nach Kamushiro, Blake, Li (1972) J. Amer. Chem. Soc. 94, 2855 erfolgen.

Nach Einführung der ersten Aminosäure muß die temporäre Schutzgruppe abgespalten werden. Dies kann z. B. acidolytisch mit Trifluoressigsäure-Methylenchlorid erfolgen. Um die während der Reaktion gebildeten t-Butylkationen, die mit nukleophilen Zentren der wachsenden Peptidkette Nebenreaktionen eingehen können, abzufangen, wird der Reaktionsmischung zweckmäßig noch 10 Vol-% Anisol zugesetzt.

$$\text{Boc-As}_1\text{-O-CH}_2\text{-Harz} \xrightarrow{\text{TFA}} \text{TFA}^- \text{H}_2^+\text{As}_1\text{-O-CH}_2\text{-Harz} + \text{CO}_2\uparrow + \text{CH}_2\text{C(CH}_3)_2$$

Wenn nach der Abspaltung der Schutzgruppe die gebundene Aminosäure in Form eines Salzes vorliegt, muß die Ammoniumgruppe zunächst in die freie Amingruppe übergeführt werden, um die nächste Kopplung ermöglichen zu können. Bei der oben beschriebenen bevorzugten Ausführungsform liegt nach der Abspaltung der Boc-Schutzgruppe durch Trifluoressigsäure das Trifluoracetylsalz der gebundenen Aminosäure vor. Um die nächste Kopplung ermöglichen zu können, muß die Ammoniumgruppe in die Form des freien Amins überführt werden. Dies geschieht mit Triäthylamin.

$$\text{TFA}^-\text{H}_2^+\text{-As}_1\text{-O-CH}_2\text{-Harz} \xrightarrow{\text{Et}_3\text{N}} \text{HAs}_1\text{-O-CH}_2\text{-Harz} + \text{TFA}^-\text{HEt}_3\text{N}^+$$

Anschließend kann die Kopplung mit der zweiten Aminosäure durchgeführt werden.

$$\text{Boc-As}_2\text{-OH} \xrightarrow[\text{HBT}]{\text{DIC}} \text{Boc-As}_2\text{-As}_1\text{-O-CH}_2\text{-Harz}$$

Die Reaktionsfolge: Abspaltung der Boc-Schutzgruppe — Freisetzung der Aminogruppe aus ihrer Salzbildung — Kopplung mit der nächsten Boc-Aminosäure wird so lange durchgeführt, bis alle Aminosäuren an das Harz gekoppelt sind.

Nach der Kopplung der Boc-geschützten Aminosäure mit den freien Aminogruppen des Harzes oder der Peptidkette wird mit dem Ninhydrintest nach Kaiser (Kaiser, E., Coloscott, R. L. Bossinger, C. C., Cook, P. L. (1970) Anal. Biochem. 34, 595–598) an einer kleinen Harzprobe untersucht, ob noch freie Aminogruppen nachweisbar sind (Blaufärbung). Wenn dies der Fall ist, muß das Harz entweder einer neuen Kopplung unterzogen werden, um die Bildung von Peptiden, denen eine Aminosäure fehlt, zu unterbinden, oder acetyliert werden. Diese Peptide wären bei der Endreinigung nur sehr schwer vom eigentlichen Produkt abtrennbar.

$$\text{HAs}_1\text{-O-CH}_2\text{-Harz} + \text{Ac}_2\text{O} \rightarrow \text{AcAs}_1\text{-O-CH}_2\text{-Harz} + \text{AcOH}$$

In der obigen allgemeinen Formel I steht das Symbol V vorzugsweise für die Aminosäuren Arg, Lys, Orn oder Pro. Das Symbol X steht vorzugsweise für die Aminosäure Gln.

Wenn das Symbol V für einen physiologisch unbedenklichen Säurerest steht, handelt es sich hierbei zweckmäßig um den Rest einer niederen aliphatischen Carbonsäure mit 1 bis 6 Kohlenstoffatomen, vorzugsweise Essigsäure und besonders vorzugsweise Propionsäure.

In den Polypeptiden der Formel I gemäß der Erfindung sind wesentlich die 5 letzten Aminosäuren, wie sie in der Formel I definiert sind. Dagegen ist es nicht wesentlich, welche Aminosäuren für die Symbole V und X stehen. Wenn V und X Aminosäuren bedeuten, handelt es sich um Heptapeptide. Wenn X eine einfache Bindung darstellt, handelt es sich um Hexapeptide. Die pharmakologische Wirkung der Verbindungen gemäß der Erfindung ist also weitgehend unabhängig davon, welche Aminosäuren für die Symbole V und/oder X stehen. Da die Verbindungen gemäß der Erfindung zweckmäßig eine gewisse Wasserlöslichkeit besitzen, um sie vorteilhaft pharmakologisch anwenden zu können, sollten die Aminosäuren, für die die Symbole V und X stehen, nicht beide sehr unpolar sein. Die oben genannten bevorzugten Aminosäuren für die Symbole sind unter diesem Gesichtspunkt bevorzugt. Der Fachmann kann leicht andere Aminosäuren einsetzen die ebenfalls eine gewisse Polarität aufweisen und zur Folge haben, daß die Polypeptide gemäß der Erfindung eine genügende Wasserlöslichkeit besitzen. Wegen der leichteren Zugänglichkeit werden in der Natur vorkommende Aminosäuren gemäß der Erfindung eingesetzt. Grundsätzlich ist es aber auch möglich nur synthetisch zugängliche Aminosäuren zu verwenden.

Gegenstand der Erfindung ist also auch die Verwendung der genannten Polypeptide oder deren pharmazeutisch annehmbare Salze bei der Bekämpfung von, insbesondere chronischen, Schmerzzuständen und Bluthochdruckerkrankungen. Dazu gehören z. B. auch chronische Entzündung der Hornhaut des Auges, die durch verschiedene Umstände hervorgerufen sein können, z. B. durch andauernde Aussetzung gegenüber UV-Licht, IR-Strahlung oder chemische Verbindungen.

Weiterhin sind Gegenstand der Erfindung pharmazeutische Präparate, die ein oder mehrere der oben genannten Peptide oder deren pharmazeutisch annehmbare Salze zusammen mit üblichen pharmazeutischen Träger- und/oder Hilfsstoffen enthalten.

Die Polypeptide gemäß der Erfindung besitzen eine außerordentlich ausgeprägte antagonistische Eigenschaft ohne erkennbare agonistische Wirkung gegenüber Substanz P.

Die in der Beschreibung verwendeten Abkürzungen haben die folgende Bedeutung:

Abkürzungen

| | |
|---|---|
| 1-HBT | 1-Hydroxybenzotriazol |
| DIC | Diisopropylcarbodiimid |
| TFA | Trifluoressigsäure |
| $Et_3N$ | Triäthylamin |
| Boc | t-Butyloxycarbonyl |
| For | Formyl |
| DMF | Dimethylformamid |
| MeOH | Methanol |
| Nle | Norleucin |
| Met | Methionin |
| Leu | Leucin |
| Trp | Tryptophan |
| Phe | Phenylalanin |
| Gln | Glutamin |
| Pro | Prolin |
| Arg | Arginin |
| Tyr | Tyrosin |
| Ile | Isoleucin |
| Val | Valin |
| L-p-Cl-Phe | p-Chlorphenylalanin |
| Lys | Lysin |
| Orn | Ornithin |

Gegenstand der Erfindung ist weiterhin ein Verfahren zur Reinigung von Polypeptiden der Formel I durch Säulenchromatographie, das dadurch gekennzeichnet ist, daß die Lösung des Polypeptids an ein Polystyrol-Ionenaustauscherharz adsorbiert und mit Wasser-acetonitril-Essigsäuregemischen mit zunehmendem Gehalt an Acetonitril eluiert wird. Als Polystyrol-Ionenaustauscherharze können die im Handel erhältlichen entsprechenden Harze eingesetzt werden, wie sie u. a. in den Beispielen genannt sind. Die Adsorption erfolgt mit einer Lösung des Polypeptids, zweckmäßig in wäßriger Essigsäure, wobei der Gehalt an Essigsäure zweckmäßig etwa 20 bis 80 Gew.-%, vorzugsweise 40 bis 60 und besonders bevorzugt etwa 50 Gew.-% beträgt. Vor der Adsorption wird die Säule aus dem Austauscherharz zweckmäßig mit Essigsäure äquilibriert, wobei deren Konzentration in diesem Fall zweckmäßig im Bereich von etwa 2 bis 40, vorzugsweise 2 bis 20 und besonders bevorzugt 4 bis 8 Gew.-% liegt. Dann wird mit Wasser-Acetonitril-Essigsäuregemischen eluiert, wobei zunächst zweckmäßig nur mit wäßriger Essigsäure eluiert wird. Dann wird mit Wasser-Acetonitril-Essigsäuregemischen mit ansteigendem Acetonitrilgehalt eluiert. Der Anteil an Essigsäure und gegebenenfalls Acetonitril in diesen wäßrigen Gemischen beträgt zweckmäßig 2 bis 20 Gew.-%, vorzugsweise etwa 4 bis 8 Gew.-%. Es kann eine graduelle oder kontinuierliche Elution vorgenommen werden. Bei der graduellen Elution wird der Gehalt an Acetonitril schrittweise, bei der kontinuierlichen Elution kontinuierlich erhöht. Die jeweils zweckmäßigen Verhältnisse zwischen Acetonitril und Essigsäure können je nach der zu reinigenden Aminosäure durch einfache Versuche ermittelt werden.

Anhand der nachfolgenden Beispiele wird die Erfindung weiter erläutert.

Bei den eingesetzten Aminosäuren und den in der Formel mit den Abkürzungen bezeichneten Aminosäuren handelt es sich jeweils um die L-Konfiguration, soweit nichts anderes angegeben ist.

Beispiel 1

A) Kopplung der ersten Aminosäure an das Harz (F. Gisin (1973) Helv. Chim. Acta 56, 1976).

2,5 g Boc-Met-OH und 1,63 g $Cs_2CO_3$ wurden in 25 ml $H_2O$ gelöst. Die Lösung wurde zur Trockne eingeengt und im Vakuum getrocknet. Der Rückstand wurde in 30 ml DMF gelöst, mit 5 g chlormethyliertem Polystyrolharz (1% quervernetzt, 1 meq Cl/g Harz) versetzt und 4 Tage bei Raumtemperatur gerührt. Danach wurde vom Lösungsmittel abfiltriert und das Harz 3 × mit je 50 ml DMF, 2 × mit 50 ml MeOH, 2 × mit 50 ml DMF, 1 × mit 50 ml MeOH und 2 × mit je 50 ml Diäthyläther gewaschen.

Nach dem Trocknen wurden 5,83 g Boc-Met-O-$CH_2$-Harz erhalten. Der Substitutionsgrad betrug 77%. Um Boc-Nle-OH an das Harz zu koppeln, wurde analog verfahren.

Im folgenden werden die einzelnen Schritte, die mit dem Harz in ein- und derselben manuellen Peptidsyntheseapparatur durchgeführt wurden, ausführlich dargestellt. Die Mengen sind für 5 g Harz angegeben.

a) Neutralisation

| Lösungsmittel/Reagenz | Volumen (ml) | Rührzeit (min) | Vorgang |
|---|---|---|---|
| $CH_2Cl_2$ | 40 | 3 | Waschen |
| $CH_2Cl_2/Et_3N$ = 9/1 | 50 | 5 | Neutralisierung |
| $CH_2Cl_2$ | 40 | 3 | Waschen |

b) Kopplung der Aminosäure

| Lösungsmittel/Reagenz | Volumen (ml) | Rührzeit (min) | Vorgang |
|---|---|---|---|
| $CH_2Cl_2$ | 40 | 3 | Waschen |
| 15 mmol Boc-Aminosäure + 15 mmol 1-HBT + 15 mmol DIC in 40 ml DMF | 40 | 180 | Kopplung |
| DMF (3 × wiederholen) | 40 | 3 | Waschen |
| $CH_2Cl_2$ (3 × wiederholen) | 40 | 3 | Waschen |
| 2-PrOH (3 × wiederholen) | 40 | 3 | Waschen |
| $CH_2Cl_2$ | 40 | 3 | Waschen |

c) Abspaltung der Boc-Schutzgruppe

| Lösungsmittel/Reagenz | Volumen (ml) | Rührzeit (min) | Vorgang |
|---|---|---|---|
| $CH_2Cl_2$ | 40 | 3 | Waschen |
| TFA/$CH_2Cl_2$/Anisol = 45/45/10 | 40 | 1,5 | Deprotektion |
| TFA/$CH_2Cl_2$/Anisol = 45/45/10 | 40 | 1,5 | Deprotektion |
| TFA/$CH_2Cl_2$/Anisol = 45/45/10 | 40 | 15 | Deprotektion |

Fortsetzung

| Lösungsmittel/Reagenz | Volumen (ml) | Rührzeit (min) | Vorgang |
|---|---|---|---|
| $CH_2Cl_2$ (3 × wiederholen) | 40 | 3 | Waschen |
| 2-PrOH (3 × wiederholen) | 40 | 3 | Waschen |
| $CH_2Cl_2$ (3 × wiederholen) | 40 | 3 | Waschen |

d) Acetylierung des Harzes

| Lösungsmittel/Reagenz | Volumen (ml) | Rührzeit (min) | Vorgang |
|---|---|---|---|
| $CH_2Cl_2$ | 40 | 3 | Waschen |
| $CH_2Cl_2/Ac_2O$/Pyridin = 80/10/10 | 50 | 3 | Kopplung |
| $CH_2Cl_2/Ac_2$/Pyridin = 80/10/10 | 50 | 20 | Kopplung |
| $CH_2Cl_2$ (3 × wiederholen) | 40 | 3 | Waschen |
| 2-PrOH (3 × wiederholen) | 40 | 3 | Waschen |
| $CH_2Cl_2$ (3 × wiederholen) | 40 | 3 | Waschen |

B) Synthese des Peptideharzes Arg-Gln-D-Trp-Phe-D-Trp-Leu-Met-$NH_2$

a) Die Synthese dieses Peptides wurde erreicht durch das Hintereinanderschalten der im vorausgehenden Abschnitt beschriebenen Reaktionsschritte in der in der folgenden Tabelle angegebenen Reaktionsfolge. Die Synthese wurde gestartet mit 5 g polychlormethyliertem Harz 0,77 meq Boc-Met/g Harz:

| Reaktionsschritt | Verbindung | Reaktionsfolge |
|---|---|---|
| 1 | Boc-Leu-Met-O-Harz | c-a-b-d |
| 2 | Boc-D-Trp-Leu-Met-O-Harz | c-a-b-d |
| 3 | Boc-Phe-D-Trp-Leu-Met-O-Harz | c-a-b-d |
| 4 | Boc-D-Trp-Phe-D-Trp-Leu-Met-O-Harz | c-a-b-d |
| 5 | Boc-Gln-D-Trp-Phe-D-Trp-Leu-Met-O-Harz | c-a-b-d |
| von diesem Harz (insgesamt 8,4 g) wurden 1,68 g weiterverarbeitet | | |
| 6 | Boc-Arg-Gln-D-Trp-Phe-D-Trp-Leu-Met-O-Harz | c-a-b |

b) Ammonolyse und Deprotektion

2,0 g Boc-Arg(HCl)-Gln-D-Trp-Phe-D-Trp-Leu-Met-O-Harz wurden in 15 ml Methanol, das mit Ammoniak gesättigt war, suspendiert. Die Mischung wurde 48 h bei Raumtemperatur gerührt und

anschließend filtriert und das Filtrat eingedampft. Das resultierende Öl wurde mit 50 ml Essigsäureäthylester verrieben und über Nacht bei 0° stehengelassen. Der Niederschlag wurde abfiltriert (750 mg). Dieses Material wurde in einer Mischung aus 10 ml DMF, 5 ml 10 N HCl und 5 ml $H_2O$ gelöst und 2 h bei Raumtemperatur gerührt. Die Lösung wurde eingedampft, in Wasser-Essigsäure aufgenommen und lyophilisiert.

c) Säulenchromatographie an Servachrom XAD-2 (eingetragenes Warenzeichen)
Eine Säule aus Servachrom XAD-2, 100—120 mesh (24 × 2,5 cm) wurde mit 6% AcOH äquilibriert. Die Substanz (etwa 600 mg) wurde in 30% AcOH (5 ml) gelöst und auf die Säule aufgetragen. Dann wurde mit einem Gemisch aus 470 ml $H_2O$, 30 ml AcOH und 88 ml $CH_3CN$ chromatographiert. Die Fließrate betrug 0,4 ml/min, die Fraktionsgröße 10 ml. Die gewünschte Substanz wurde in den Fraktionen 51—105 eluiert. Diese wurden gesammelt, eingedampft und lyophilisiert.
Ausbeute: 160 mg
(120 μmol, 15% d. Th. bez. auf Boc-Met-Gehalt des Harzes).

Analyse von H-Arg-Gln-D-Trp-Phe-D-Trp-Leu-Met-$NH_2$

HPLC

Die Substanz zeigte einen einzigen symmetrischen Peak in dem folgenden HPLC-System:

Säule: μ-Bondapak C18
Fließmittel: $CH_3CN$-K-Phosphatpuffer pH 3,0 0,1$^M$ = 35—65
Fließrate: 1,5 ml/min
Retentionszeit: 8,33 min

Dünnschichtchromatographie

Die Substanz zeigte in den folgenden Fließmittelsystemen einen einzigen, symmetrischen Peak (Träger: Kieselgel 60): Laufstrecke: 15 cm.

| | $R_f$ |
|---|---|
| 2-PrOH-1$^M$ AcOH = 2 : 1 | 0 |
| EtOAc-Py-AcOH-$H_2O$ = 60 : 20 : 6 : 11 | 0,115 |
| n-BuOH-AcOH-$H_2O$ = 4 : 1 : 1 | 0,401 |
| EtOAc-Py-AcOH-$H_2O$ = 120 : 20 : 6 : 11 | 0 |

Aminosäureanalyse

Etwa 1 mg Peptid wurde bei 110° 24 h in 1 ml 6 N HCl hydrolisiert. Es wurden die folgenden Ergebnisse erhalten:

Glu 1,03 (1), Met 1,0 (1), Leu 0,94 (1), Phe 0,97 (1), Arg 1,06 (1).

Beispiel 2 und 3

H-Pro-Gln-D-Trp-Phe-D-Trp-Leu-Met-$NH_2$
Pr-Gln-D-Trp-Phe-D-Trp-Leu-Met-$NH_2$

a) Die Synthese dieser Peptide wurde erreicht durch das Hintereinanderschalten der in Beispiel 1 genauer beschriebenen Reaktionsschritte in der in der Tabelle angegebenen Reaktionsfolge. Die Synthese wurde gestartet mit 5,83 g Boc-Met-O-Harz (0,77 meq Boc-Met/g Harz).

| Reaktionsschritt | Verbindung | Reaktionsfolge |
|---|---|---|
| 1 | Boc-Leu-Met-O-Harz | c-a-b |
| 2 | Boc-D-Trp(For)-Leu-Met-O-Harz | c-a-b |
| 3 | Boc-Phe-D-Trp(For)-Leu-Met-O-Harz | c-a-b |

Fortsetzung

| Reaktionsschritt | Verbindung | Reaktionsfolge |
|---|---|---|
| 4 | Boc-D-Trp(For)-Phe-D-Trp(For)-Leu-Met-O-Harz | c-a-b |
| 5 | Boc-Gln-D-Trp(For)-Phe-D-Trp(For)-Leu-Met-O-Harz | c-a-b |

Von diesem Peptidharz (8,74 g) wurden je 2,18 g für die Synthese der beiden Endprodukte verwendet.

| | | |
|---|---|---|
| 6 a | Boc-Pro-Gln-D-Trp(For)-Phe-D-Trp(For)-Leu-Met-O-Harz | c-a-b |
| 7 a | TFA · Pro-Gln-D-Trp(For)-Phe-D-Trp(For)-Leu-Met-O-Harz | c |
| 6 b | Pr-Gln-D-Trp(For)-Phe-D-Trp(For)-Leu-Met-O-Harz | c-a-b |

b) Ammonolyse von H-Pro-Gln-D-Trp(For)-Phe-D-Trp(For)-Leu-Met-O-$CH_2$-Harz

Das Harz (2,33 g) wurde mit 20 ml Methanol, das vorher bei 0° mit gasförmigem Ammoniak gesättigt worden war, übergossen und 24 h bei Raumtemperatur in einem gut verschlossenen Rundkolben gerührt. Anschließend wurde die Suspension filtriert und das Harz 5 × mit je 50 ml Methanol gewaschen. Das Gewicht des getrockneten Harzes betrug dann 1,3 g. Das klare Filtrat wurde eingedampft und der ölige Rückstand i.V. getrocknet.
Gewicht: 1,06 g.

c) Säulenchromatographische Reinigung von H-Pro-Gln-D-Trp-Phe-D-Trp-Leu-Met-$NH_2$

Das Produkt aus b) wurde, gelöst in 10 ml 50%iger AcOH an einer Säule aus Servachrom XAD-2 (100–120 mesh, 2,5 × 45 cm), die vorher mit 6%iger Essigsäure äquilibriert worden war, aufgetragen. Es wurde nacheinander mit den folgenden Fließmitteln eluiert:

1. 400 ml 6% AcOH
2. 400 ml 6% AcOH—$CH_3CN$ = 9 : 1
3. 400 ml 6% AcOH—$CH_3CN$ = 8 : 2
4. 400 ml 6% AcOH—$CH_3CN$ = 7 : 3
5. 800 ml 6% AcOH—$CH_3CN$ = 6 : 4

Die Fraktionsgröße betrug 10 ml, die Fließrate 10 ml/25 min. Das Produkt wurde in den Fraktionen 251–271 eluiert. Die Lösung wurde eingedampft, der Rückstand in n-BuOH/Wasser aufgenommen und lyophilisiert.

d) Analytik

HPLC: Das Produkt zeigt einen einzigen symmetrischen Peak bei der Chromatographie in dem folgenden System:

Säule: $\mu$-Bondapak $C_{18}$ (Fa. Waters) (2,4 × 300 mm)
Fließmittel: $CH_3CN$ (40%)-0,1 M K-Phosphatpuffer pH 3,0 (60%)
Die beobachtete Retentionszeit betrug 15,8 min (Fließrate 1,5 ml/min)

DC: Die Substanz zeigte einen einzigen symmetrischen Fleck in 4 verschiedenen Systemen. Als feste Phase dienten Merck-Fertigplatten Kieselgel 60 (5 × 20 cm)

| | $R_f$ |
|---|---|
| n-BuOH-AcOH-$H_2O$ = 4 : 1 : 1 | 0,64 |
| 2-PrOH-1 M AcOH = 2 : 1 | 0,191 |
| EtOAc-Py-AcOH-$H_2O$ = 60 : 20 : 6 : 11 | 0,51 |
| EtOAc-Py-AcOH-$H_2O$ = 120 : 20 : 6 : 11 | 0,19 |

Aminosäureanalyse

Das Peptid (etwa 1 mg) wurde in 1 ml 4 m Methansulfonsäure bei 120°C 24 h hydrolysiert. Folgende Werte wurden erhalten:

Glu 1,06 (1), Pro 1,07 (1), Met 0,94 (1), Leu 0,99 (1), Phe 0,92 (1), $NH_3$ 1,96 (2), Trp 2,05 (2).

e) Ammonolyse von Propionyl-Gln-D-Trp(For)-Phe-D-Trp(For)-Leu-Met-O-$CH_2$-Harz

Das Harz (2,15 g) wurde mit 20 ml Methanol, das vorher bei 0° mit gasförmigem Ammoniak gesättigt worden war, in einem gut verschlossenen Rundkolben 24 h gerührt. Anschließend wurde die Suspension filtriert und das Harz 5 × mit 30 ml Methanol gewaschen. Das Gewicht des Harzes betrug dann 1,22 g. Das klare Filtrat wurde eingedampft und der ölige Rückstand i.V. getrocknet. Gewicht: 900 mg.

f) Säulenchromatographische Reinigung
Propionyl-Gln-D-Trp-Phe-D-Trp-Leu-Met-$NH_2$

Das Produkt wurde in 10 ml 50% AcOH gelöst und auf eine Säule aus Servachrom XAD-2 (100–120 mesh, 2,5 × 45 cm), die vorher mit 6%iger Essigsäure äquilibiert worden war, aufgetragen. Es wurde nacheinander mit den folgenden Fließmitteln eluiert:

1. 400 ml 6% AcOH
2. 400 ml 6% AcOH-$CH_3CN$ = 9 : 1
3. 400 ml 6% AcOH-$CH_3CN$ = 8 : 2
4. 400 ml 6% AcOH-$CH_3CN$ = 7 : 3
5. 800 ml 6% AcOH-$CH_3CN$ = 6 : 4

Die Fließrate betrug 10 ml/25 min, die Fraktionsgröße 10 ml. Das Produkt wurde in den Fraktionen 171–189 eluiert. Die Lösung wurde lyophilisiert.
Ausbeute: 93 mg (chromatographisch reines Produkt) + 240 mg (chromatographisch leicht verunreinigt).

g) Analytik:

HPLC: Es wurden unter denselben Bedingungen gearbeitet wie oben beschrieben. Die Substanz zeigte einen einzelnen symmetrischen Peak mit einer Retentionszeit von 18 min.
DC (Dünnschichtchromatographie): Die Substanz zeigte einen einzelnen symmetrischen Fleck in vier verschiedenen Fließmittelsystemen:

| | $R_f$ |
|---|---|
| n-BuOH-AcOH-$H_2O$ = 4 : 1 : 1 | 0,78 |
| 2-PrOH-1 M AcOH = 2 : 1 | 0,55 |
| EtOAc-Py-AcOH-$H_2O$ = 60 : 20 : 6 : 1 | 0,76 |
| EtOAc-Py-AcOH-$H_2O$ = 120 : 20 : 6 : 1 | 0,4 |

Pharmakologische Untersuchungen

Material und Methode

Meerschweinchen Ileum wurde nach dem Töten der Tiere herausgeschnitten, durch Ziehen am caudalen Ende von Mesenterien weitgehend befreit und mit Tyrode-Lösung von Verdauungsprodukten befreit. Vom caudalen Ende wurden ca. 25 cm verworfen und daran anschließend mehrere 4 cm lange Darmabschnitte abgetrennt und in Tyrode-Lösung aufbewahrt. Die Tyrode-Lösung wurde mit Carbogen (95% $O_2$, 5% $CO_2$) begast. Die Tyrode-Lösung enthielt pro Liter folgende Substanzen:

8 g NaCl; 2 g KCl; 1,3 g $CaCl_2 \cdot 2\,H_2O$;
2,1 g $MgCl_2 \cdot 6\,H_2O$; 10 g $NaHCO_3$; 0,58 g $NaH_2PO_4 \cdot 1\,H_2O$;
11 g D(+)-Glucose.

Die Tyrode-Lösung wurde in einem Organbad auf 38°C temperiert. Jedes Präparat behielt seine volle Kontraktionsfähigkeit für 4 bis 7 Stunden. Darmabschnitte von 4 cm wurden auf beiden Seiten abgebunden und im Organbad aufgehängt. Die Kontraktionen des Präparates wurden unter isotonischen

Meßbedingungen über einen Hebel auf einer Kymographen-Trommel aufgezeichnet. Zum Schreiben diente ein Filzschreiber, der als Seitenschreiber angebracht war. Der Lastarm war 225 mm, der Kraftarm 20 mm lang. Die Vorlast betrug ca. 0,3 g, der Papiervorschub 5 mm/min. Das Organbad faßte 10 ml.

In der Regel wurden die Testsubstanzen in Volumina von 20 bis 10 μl zugesetzt und durch die Begasung augenblicklich im Organbad verteilt. Alle zugesetzten Testsubstanzen wurden in Tyrode-Lösung gelöst bzw. mit Tyrode-Lösung aus einer Stammlösung verdünnt.

Testsubstanzen: als Agonisten dienten Substanzen P (Serva) oder Norleucin-SP (Ferring, Kiel) in Dosen zwischen $10^{-9}$ und $10^{-7}$ M. Als pontentielle Antagonisten wurden getestet:

Verbindung 1: D-$Pro^2$, D-$Trp^{7,9}$-SP
Verbindung 2: $Arg^5$, D-$Trp^{7,9}$-$SP_{5-11}$
Verbindung 3: $Pro^5$, D-$Trp^{7,9}$-$SP_{5-11}$
Verbindung 4: Propionyl D-$Trp^{7,9}$-$SP_{6-11}$

Im Standard-Test wurde SP bzw. Nle-SP in einer Konzentration zwischen $10^{-9}$ und $10^{-7}$ M appliziert, nach ca. 2 min ausgewaschen. Nach einer Latenzzeit von weiteren 3 min wurder der potentielle Antagonist in einer Konzentration von $3 \times 10^{-6}$, $10^{-5}$ oder $3,3 \times 10^{-5}$ M appliziert, direkt gefolgt von einer weiteren Dosis SP (Nle-SP). Nach erneutem Auswaschen und einer Wartezeit von 3 min wurde wiederum SP (Nle-SP) in der gleichen Dosis verabreicht, um zu prüfen, ob die ursprüngliche Kontraktionsstärke erreicht wird.

Die Verminderung der relativen Kontraktion der Ileum Präparate als Folge der Anwendung von SP-Analogen wurde als Maß für die relative Wirksamkeit als Antagonisten angesehen.

## Ergebnisse

Alle getesteten Analoge von Substanz-P: Verbindungen 1, 2, 3 und 4 besitzen Substanz-P-antagonisierende Wirkung, wenn sie in einer Konzentration von $10^{-5}$ M eingesetzt werden. Dabei ergibt sich eine Abhängigkeit von der Dosis des Agonisten (SP oder Nle-SP). Im unteren Dosisbereich sind die Antagonisten stärker wirksam als im oberen Bereich.

Zum Vergleich der Wirksamkeit zwischen den verschiedenen Antagonisten sind in der Tabelle ihre Hemmwirkungen dargestellt, wenn der Agonist in einer Konzentration von $10^{-8}$ M, der Antagonist $10^{-5}$ M vorliegt.

| Agonist | Antagonist | prozentuale Hemmwirkung | Zahl der Experimente |
|---|---|---|---|
| SP oder Nle-SP $10^{-8}$ M | Verb. 1 (AP 2) $10^{-5}$ M | $67,0 \pm 3$ | 2 |
| | Verb. 2 (AP 4) $10^{-5}$ M | $68,1 \pm 8$ | 7 |
| | Verb. 3 (AP 5/2) $10^{-5}$ M | $69,5 \pm 0,5$ | 2 |
| | Verb. 4 (AP 6) $10^{-5}$ M | $29,3 \pm 9,5$ | 3 |

Nach den vorliegenden Befunden sind die Verbindungen 1, 2 und 3 am stärksten wirksam. Ihre antagonistische Potenz ist etwa gleich stark.

Niedrige, jedoch bedeutende Hemmwirkung, besitzt dagegen Verbindung 4, das um eine Aminosäure kürzere Peptid.

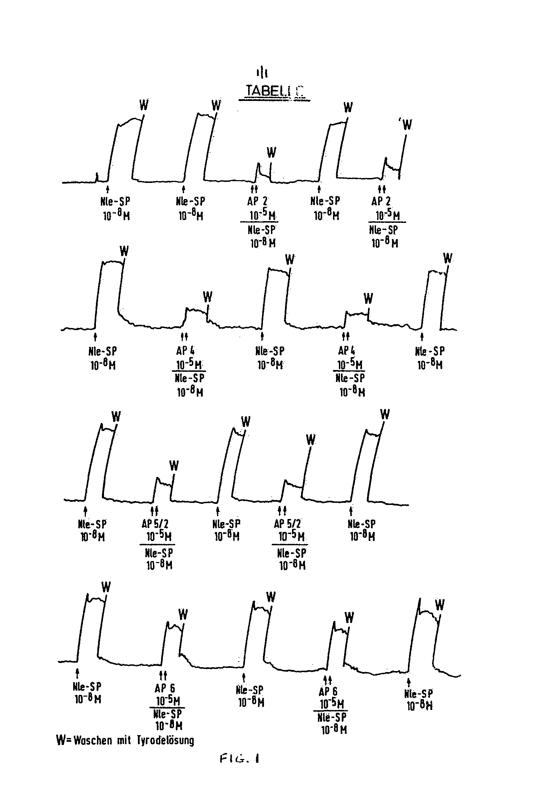

Originaldarstellungen der isotonischen Meßdaten finden sich in der Abbildung 1.

**Patentansprüche für die Vertragsstaaten: CH, DE, FR, GB, IT, LI, NL, SE**

1. Polypeptide der Formel I

V-X-D-Trp-Y-D-Trp-Leu-Z-$NH_2$

wobei

V eine in der Natur vorkommende Aminosäure oder einen physiologisch unbedenklichen Säurerest,
X eine in der Natur vorkommende Aminosäure oder eine einfache Bindung,

Y eine der Aminosäuren, Phe, Ile, Val, Tyr und L-p-Cl-Phe, und
Z die Aminosäuren Met und Nle

darstellen und deren Salze.

2. Polypeptide nach Anspruch 1, dadurch gekennzeichnet, daß das Symbol V für eine der Aminosäuren Arg, Lys, Orn oder Pro steht.

3. Polypeptide nach Anspruch 1 oder 2, dadurch gekennzeichnet, daß das Symbol X für die Aminosäure Gln steht.

4. Verfahren zur Herstellung der Polypeptide der Formel

$V\text{-}X\text{-}D\text{-}Trp\text{-}Y\text{-}D\text{-}Trp\text{-}Leu\text{-}Z\text{-}NH_2$

wobei

V eine in der Natur vorkommende Aminosäure oder einen physiologisch unbedenklichen Säurerest,
X eine in der Natur vorkommende Aminosäure oder eine einfache Bindung,
Y eine der Aminosäuren Phe, Ile, Val, Tyr und L-p-Cl-Phe, und
Z die Aminosäuren Met und Nle

darstellen und deren Salze, dadurch gekennzeichnet, daß man in an sich bekannter Weise nach der Festphasenmethode an ein Trägerharz zuerst die C-terminale Aminosäure und dann daran die anderen Aminosäuren oder, falls V einen physiologisch unbedenklichen Säurerest darstellt, die physiologisch unbedenkliche Säure schrittweise koppelt und das Trägerharz abspaltet.

5. Pharmazeutische Präparate, dadurch gekennzeichnet, daß sie ein oder mehrere Polypeptide oder deren pharmazeutisch annehmbare Salze nach Anspruch 1 bis 4 zusammen mit üblichen pharmazeutischen Trägerstoffen und/oder Hilfsstoffen enthalten.

6. Verwendung der Polypeptide der Formel I oder deren pharmazeutisch annehmbaren Salze nach Ansprüchen 1 bis 4 als Pharmazeutikum.

7. Verwendung der Polypeptide der Formel I oder deren pharmazeutisch annehmbaren Salze nach Anspruch 1 bis 4 bei der Bekämpfung von, insbesondere chronischen, Schmerzzuständen, Entzündungen und Bluthochdruckerkrankungen.

8. Verfahren zur Reinigung von Polypeptiden der Formel I nach Anspruch 1 durch Säulenchromatographie, dadurch gekennzeichnet, daß die Lösung des Polypeptids an Polystyrolharz adsorbiert und mit Wasser-Acetonitril-Essigsäuregemischen mit zunehmendem Gehalt an Acetonitril eluiert wird.

**Patentansprüche für den Vertragsstaat: AT**

1. Verfahren zur Herstellung der Polypeptide der Formel

$V\text{-}X\text{-}D\text{-}Trp\text{-}Y\text{-}D\text{-}Trp\text{-}Leu\text{-}Z\text{-}NH_2$

wobei

V eine in der Natur vorkommende Aminosäure oder einen physiologisch unbedenklichen Säurerest,
X eine in der Natur vorkommende Aminosäure oder eine einfache Bindung,
Y eine der Aminosäuren Phe, Ile, Val, Tyr und L-p-Cl-Phe, und
Z die Aminosäuren Met und Nle

darstellen und deren Salze, dadurch gekennzeichnet, daß man in an sich bekannter Weise nach der Festphasenmethode an ein Trägerharz zuerst die C-terminale Aminosäure und dann daran die anderen Aminosäuren oder, falls V einen physiologisch unbedenklichen Säurerest darstellt, die physiologisch unbedenkliche Säure schrittweise koppelt und das Trägerharz abspaltet.

2. Verfahren nach Anspruch 1, dadurch gekennzeichnet, daß das Symbol V für eine der Aminosäuren Arg, Lys, Orn oder Pro steht.

3. Verfahren nach Anspruch 1 oder 2, dadurch gekennzeichnet, daß das Symbol X für die Aminosäure Gln steht.

4. Verfahren zur Reinigung von Polypeptiden der Formel I nach Anspruch 1 durch Säulenchromatographie, dadurch gekennzeichnet, daß die Lösung des Polypeptids an Polystyrolharz adsorbiert und mit Wasser-Acetonitril-Essigsäuregemischen mit zunehmendem Gehalt an Acetonitril eluiert wird.

**Claims for the Contracting States: CH/LI, DE, FR, GB, IT, NL, SE**

1. Polypeptides of the formula I

$V\text{-}X\text{-}D\text{-}Trp\text{-}Y\text{-}D\text{-}Trp\text{-}Leu\text{-}Z\text{-}NH_2$

wherein

V represents an amino acid occuring in nature or a physiologically acceptable acid residue,
X represents an amino acid occuring in nature or a single bond,
Y represents one of the amino acids Phe, Ile, Val, Tyr or L-p-Cl-Phe and
Z represents the amino acids Met or Nle,

and salts thereof.

2. Polypeptides according to claim 1, characterized in that the symbol V represents one of the amino acids Arg, Lys, Orn or Pro.

3. Polypeptides according to claim 1 or 2, characterized in that the symbol X represents the amino acid Gln.

4. Process for preparing the polypeptides of the formula

$V\text{-}X\text{-}D\text{-}Trp\text{-}Y\text{-}D\text{-}Trp\text{-}Leu\text{-}Z\text{-}NH_2$

wherein

V represents an amino acid occuring in nature or a physiologically acceptable acid residue,
X represents an amino acid occuring in nature or a single bond,
Y represents one of the amino acids Phe, Ile, Val, Tyr or L-p-Cl-Phe and
Z represents the amino acids Met or Nle,

and salts thereof, characterized in that in a manner known per se by solid face method there are coupled stepwise to a carrier resin the C-terminal amino acid first and then thereto the other amino acids or, if V is a physiologically acceptable acid residue, the physiologically acceptable acid and the carrier resin is split off.

5. Pharmaceutical preparations, characterized in that they contain one or more polypeptides of pharmaceutically acceptable salts thereof according to claims 1 to 4, together with usual pharmaceutical excipients and/or auxiliaries.

6. Use of the polypeptides of the formula I or pharmaceutically acceptable salts thereof according to claims 1 to 4 as a pharmaceutical.

7. Use of the polypeptides of the formula I or pharmaceutically acceptable salts thereof according to claims 1 to 4 in combating pain conditions, inflammations und hypertension diseases, in particular chronic cases thereof.

8. Process for purifying the polypeptides of formula I according to claim 1 by column chromatography, characterized in that the solution of the polypeptide is adsorbed onto polystyrene resin and eluted with water/acetonitrile/acetic acid mixtures with an increasing content of acetonitrile.

**Claims for the Contracting State: AT**

1. Process for preparing the polypeptides of the formula

$V\text{-}X\text{-}D\text{-}Trp\text{-}Y\text{-}D\text{-}Trp\text{-}Leu\text{-}Z\text{-}NH_2$

wherein

V represents an amino acid occuring in nature or a physiologically acceptable acid residue,
X represents an amino acid occuring in nature or a single bond,
Y represents one of the amino acids Phe, Ile, Val, Tyr or L-p-Cl-Phe and
Z represents the amino acids Met or Nle,

and salts thereof, characterized in that in a manner known per se by solid face method there are coupled stepwise to a carrier resin the C-terminal amino acid first and then thereto the other amino acids or, if V is a physiologically acceptable acid residue, the physiologically acceptable acid and the carrier resin is split off.

2. Process according to claim 1, characterized in that the symbol V represents one of the amino acids Arg, Lys, Orn or Pro.

3. Process according to claim 1 or 2, characterized in that the symbol X represents the amino acid Gln.
4. Process for purifying the polypeptides of the formula I according to claim 1 by column chromatography, characterized in that the solution of the polypeptide is absorbed onto polystyrene resin and eluted with water/acetonitrile/acetic acid mixtures with an increasing content of acetonitrile.

**Revendications pour les Etats contractants: CH/LI, DE, FR, GB, IT, NL, SE**

1. Polypeptides de la formule I

V-X-D-Trp-Y-D-Trp-Leu-Z-$NH_2$

où

V représente un acide aminé présent dans la nature ou un reste acide physiologiquement inoffensif
X un acide aminé présent dans la nature ou une simple liaison
Y l'un des acides aminés Phe, Ile, Val, Tyr ou L-p-Cl-Phe, et
Z les acides aminés Met et Nle

et leurs sels.
2. Polypeptides selon la revendication 1, caractérisés par le fait que le symbole V représente l'un des acides aminés Arg, Lys, Orn ou Pro.
3. Polypeptides selon la revendication 1 ou 2, caractérisés par le fait que le symbole X représente l'acide aminé Gln.
4. Procédé de préparation des polypeptides de la formule

V-X-D-Trp-Y-D-Trp-Leu-Z-$NH_2$

où

V représente un acide aminé présent dans la nature ou un reste acide physiologiquement inoffensif,
X un acide aminé présent dans la nature ou une simple liaison,
Y l'un des acides aminés Phe, Ile, Val, Tyr et L-p-Cl-Phe, et
Z les acides aminés Met et Ile

et leurs sels, caractérisé en ce fait que, par un moyen connu en soi, selon la méthode en phase solide, on fixe par couplage sur une résine support tout d'abord l'acide aminé C-terminal puis, successivement, les autres acides aminés ou, si V représente un reste acide physiologiquement inoffensif, l'acide physiologiquement inoffensif et on sépare la résine support.
5. Préparations pharmaceutiques caractérisées par le fait qu'elles contiennent un ou plusieurs polypeptides ou leurs sels acceptables du poits de vue pharmaceutique selon les revendications 1 à 4 avec les excipients et/ou les solvants pharmaceutiques habituels.
6. Utilisation des polypeptides de la formule I ou de leurs sels acceptables du point de vue pharmaceutique selon les revendications 1 à 4 en tant que produit pharmaceutique.
7. Utilisation des polypeptides de la formule I ou de leurs sels acceptables du point de vue pharmaceutique selon les revendications 1 à 4 pour la lutte contre les douleurs, les inflammations et les affections dues à l'hypertension, en particulier chroniques.
8. Procédé de purification des polypeptides de la formule I selon la revendication 1 par chromatographie sur colonne, caractérisé par le fait que la solution du polypeptide est adsobée sur une résine de polystyrène et est éluée avec des mélanges eau-acétonitrile-acide acétique avec une teneur croissante en acétonitrile.

**Revendications pour l'Etat contractant: AT**

1. Procédé de préparation des polypeptides de la formule

V-X-D-Trp-Y-D-Trp-Leu-Z-$NH_2$

où

V représente un acide aminé présent dans la nature ou un reste acide physiologiquement inoffensif,
X un acide aminé présent dans la nature ou une simple liaison,
Y l'un des acides aminés Phe, Ile, Val, Tyr et L-p-Cl-Phe, et
Z les acides aminés Met et Nle

et leurs sels, caractérisé en ce que, par un moyen connu en soi, selon la méthode en phase solide, on fixe par couplage sur une résine support en premier lieu l'acide aminé C-terminal puis, successivement, les autres acides aminés ou, si V représente un reste acide physiologiquement inoffensif, l'acide physiologiquement inoffensif et on sépare la résine support.

2. Procédé selon la revendication 1, caractérisé par le fait que le symbole V représente l'un des acides aminés Arg, Lys, Orn ou Pro.

3. Procédé selon la revendication 1 ou 2, caractérisé par le fait que le symbole X représente l'acide aminé Gln.

4. Procédé de purification des polypeptides de la formule I selon la revendication 1 par chromatographie sur colonne, caractérisé par le fait que la solution du polypeptide est adsorbée sur une résine de polystyrène et est éluée avec des mélanges eau-acétonitrile-acide acétique avec une teneur croissante en acétonitrile.

W
W
W
W
W
Nle-SP 10⁻⁸M
Nle-SP 10⁻⁸M
AP 2 10⁻⁵M / Nle-SP 10⁻⁸M
Nle-SP 10⁻⁸M
AP 2 10⁻⁵M / Nle-SP 10⁻⁸M
Nle-SP 10⁻⁸M
AP 4 10⁻⁵M / Nle-SP 10⁻⁸M
Nle-SP 10⁻⁸M
AP 4 10⁻⁵M / Nle-SP 10⁻⁸M
Nle-SP 10⁻⁸M
Nle-SP 10⁻⁸M
AP 5/2 10⁻⁵M / Nle-SP 10⁻⁸M
Nle-SP 10⁻⁸M
AP 5/2 10⁻⁵M / Nle-SP 10⁻⁸M
Nle-SP 10⁻⁸M
Nle-SP 10⁻⁸M
AP 6 10⁻⁵M / Nle-SP 10⁻⁸M
Nle-SP 10⁻⁸M
AP 6 10⁻⁵M / Nle-SP 10⁻⁸M
Nle-SP 10⁻⁸M
W= Waschen mit Tyrodelösung

FIG. 1